Europäisches Patentamt

European Patent Office

Office européen des brevets

(19)

(11) **EP 0 800 827 B1**

(12) **EUROPÄISCHE PATENTSCHRIFT**

(45) Veröffentlichungstag und Bekanntmachung des Hinweises auf die Patenterteilung:
**16.10.2002 Patentblatt 2002/42**

(51) Int Cl.⁷: **A61K 35/78**, A61P 7/02

(21) Anmeldenummer: **97105947.2**

(22) Anmeldetag: **10.04.1997**

(54) **Regelung der Reaktivität von humanen Blutplättchen durch Verabreichung eines Extrakts der Strandkiefer (Pycnogenol TM)**

Regulation of the reactivity of human blood platelets by administration of an extract of sea pine (pycnogenol TM)

Régulation de la réactivité des plaquettes sanguines humaines par administration d'un extrait de pin maritime (pycnogénol TM)

(84) Benannte Vertragsstaaten:
**AT CH DE DK FI FR GB IT LI SE**

(30) Priorität: **10.04.1996 US 631738**

(43) Veröffentlichungstag der Anmeldung:
**15.10.1997 Patentblatt 1997/42**

(73) Patentinhaber: **Horphag Research Limited
St. Peter Port, Guernsey, Channel Islands (GB)**

(72) Erfinder: **Rohdewald, Peter, Prof. Dr.
48341 Altenberge (DE)**

(74) Vertreter: **Huber, Bernhard, Dipl.-Chem. et al
Weickmann & Weickmann
Patentanwälte
Kopernikusstrasse 9
81679 München (DE)**

(56) Entgegenhaltungen:
**US-A- 4 698 360**

- **ARZNEIMITTEL FORSCHUNG DRUG RESEARCH., Bd. 44, Nr. 1, 1994, AULENDORF DE, Seiten 17-25, XP002037387 H. T. A. CHEUNG: "INHIBITION OF PLATELET AGGREGATION BY DITERPENE ACIDS FROM PINUS MASSONIA RESIN"**
- **BRITISH JOURNAL OF NUTRITIN, Bd. 72, 1994, Seiten 775-783, XP002037388 MICHIHIRO SUGANO ET AL.: "INFLUENCE OF KOREAN PINE (PINUS KORAIENSIS)-SEED OIL CONTAINING CIS-5, CIS-9, CIS-12 OCTADECATRIENOIC ACID ON POLYUNSATURATED FATTY ACID METABOLISM, EICOSANOID PRODUCTION AND BLOOD PRESSURE OF RATS"**

**Beschreibung**

Die vorliegende Erfindung betrifft die Verwendung eines Rindenextrakts der Strandkiefer zur Herstellung eines Mittels zur Regelung der Blutplättchenaggregation beim Menschen durch Hemmung der durch Rauchen, Thromboxan oder Adrenalin induzierten Plättchenaggregation.

Die Plättchenaggregation ist ein Faktor, der zu einer großen Anzahl von Herzgefäßerkrankungen beiträgt. Im Krankheitsverlauf von Arteriosklerose ist neben Läsionen des Endotheliums die Aktivierung der Plättchenaggregation, welche zur Anhaftung von Plättchenaggregaten an der Gefäßwand führt, einer der ersten Schritte bei der Thrombusbildung.

Folglich wird die Hemmung der Plättchenaggregation bei der Schlaganfall-Prophylaxe angewendet. Deshalb wurde Acetylsalicylsäure (ASS), ein gut bekannter Inhibitor der Plättchenaggregation, in klinischen Untersuchungen zur Prävention von arterio-thrombotischen, cerebralen oder myocardialen Infarkten erfolgreich getestet. Es wurde gefunden, daß ASS die Anzahl von Infarkten bei Freiwilligen und auch bei Risikopatienten verringert. In einer Teilpopulation von Patienten konnte eine Plättchenaggregation nicht mit ASS verhindert werden. Ein Grund für das Fehlen der anti-thrombotischen Wirkung von ASS bei diesen Patienten kann die Tatsache sein, daß Adrenalin in der Lage ist, Plättchenaggregation selbst in Gegenwart von ASS zu induzieren. Da Adrenalin unter Streß gebildet wird, besteht die Möglichkeit, daß Patienten, die nicht auf eine ASS antithrombotische Prophylaxe ansprechen, unter einem größerem ständigen Streß stehen als solche, die ansprechen.

Ein hoher Risikofaktor für Herzgefäßerkrankungen ist Rauchen. Rauchen erhöht die Plättchenreaktivität und es wurde gezeigt, daß Nikotin die Thrombusbildung induziert. Neben Nikotin oder zusätzlich zu Nikotin kann auch der Teeranteil von Rauch eine erhöhte Plättchenreaktivität erzeugen.

Es wurde gezeigt, daß ASS in der Lage ist, die nach Rauchen erhöhte Plättchenaggregation zu verringern. Jedoch konnte auch in diesem Fall ASS die Rauchinduzierte Erhöhung der Plättchenfunktion bei Menschen mit Herzkranzerkrankungen nicht verhindern.

Es scheint deshalb, daß der Wirkungsmechanismus von ASS, d.h. die irreversible Acetylierung von Cyclooxygenase, die Plättchenaggregation nicht in jedem Fall verhindern kann.

Neben diesen Fällen fehlender Prävention sind die Nebenwirkungen bei regelmäßiger Einnahme von ASS nicht unbeachtlich, selbst wenn relativ geringe Dosen verwendet werden. Magenbluten und allergische Reaktionen wie ASSinduziertes Asthma und selbst ernsthafte dermatologische Reaktionen wie das Lyell-Syndrom müssen in Betracht gezogen werden.

Wenn es möglich wäre, Plättchenaggregation durch eine Substanz zu hemmen, welche eine erhöhte Plättchenreaktivität verhindert ohne jedoch Bluten oder allergisches Asthma hervorzurufen, könnte das Nutzen/Risiko-Verhältnis bei der Infarktprophylaxe verbessert werden. Ein weiterer Vorteil könnte durch eine Substanz erzielt werden, welche die Adrenalin-induzierte Plättchenaggregation hemmt.

US-4,698,360 beschreibt die Verwendung eines Proantocyanidine enthaltenden Pflanzenextrakts zur Bekämpfung bzw. Hemmung von Auswirkungen, die freie Radikale in Menschen und Tieren hervorrufen können.

H.T.A. Cheung et al., Arzneim.-Forsch./Drug Res. 44, (1) (1994) 17-24 beschreibt die Inhibierung einer durch Plättchenaktivierungsfaktor (PAF), Adenosindiphosphat (ADP) oder Calciumionophor (A23187) induzierten Plättchenaggregation durch Diterpensäuren, die aus dem Harz von Pinus massoniana gewonnen wurden.

Es ist deshalb eine Aufgabe der vorliegenden Erfindung, ein Mittel bereitzustellen, welches in der Lage ist, eine erhöhte Plättchenreaktivität zu normalisieren, ohne die Blutungszeit als ein Maß der Blutungsneigung nachteilig zu beeinflussen. Eine weitere Aufgabe der Erfindung ist es, ein Mittel bereitzustellen, welches nicht nur die Plättchenreaktivität im gleichen Ausmaß wie ASS - getestet am Modell der durch Rauchen induzierten Plättchenreaktivität - normalisiert, sondern zusätzlich die Adrenalin-induzierte Plättchenaggregation verhindert.

Es ist eine weitere Aufgabe, ein Verfahren zur Regelung der menschlichen Blutplättchenreaktivität insbesondere durch Hemmung von Adrenalin induzierter Plättchenaggregation bereitzustellen. Diese Ziele werden durch die Verwendung eines Rindenextrakts der Strandkiefer, Pinus maritima, umfassend Procyanidine zur Herstellung eines Mittels zur Regelung der Blutplättchenaggregation beim Menschen durch Hemmung der durch Rauchen oder/und Thromboxan oder/und Adrenalin induzierten Plättchenaggregation unter normaler Oxygenierung im. Gewebe erreicht, wobei ein Mittel zur Verabreichung von 200 bis 500 mg des Rindenextrakts der Strandkiefer pro Tag hergestellt wird. Der Extrakt der Strandkieferrinde wird im folgenden als Pycnogenol® bezeichnet. Pycnogenol® enthält Procyanidine, bestehend aus Katechin und Epikatechineinheiten, die durch C-C-Bindungen verknüpft sind, um Dimere, Trimere und andere Oligomere bis zu einer Kettenlänge von 6 bis 7 Moleküle zu bilden und Phenolsäuren und deren Glucosederivate (Blazsó, G., Gábor, M., Sibbel, R. und Rohdewald, P.: Antiinflammatory and superoxide radical scavenging activities of a procyanidins containing extract from the bark of Pinus pinaster Sol. and its fractions, Pharm. Pharmacol. Lett. 3, 217 (1994)). Pycnogenol® wird gemäß US-PS-4,698,360 hergestellt. Der erfindungsgemäß verwendete Extrakt kann im wesentlichen durch Extrahieren von Strandkieferrinde in zerkleinerter Form mit kochendem Wasser, Sättigen des gefilterten Extrakts mit Natriumchlorid oder, alternativ, Zugabe von Ammoniumsulfat auf 20 % w/v, Abtrennen des gebildeten Präzipitats, wiederholtes Extrahieren des Überstandes mit 1/10 Volumen Ethylacetat, Trocknen des gesammelten Ethylacetatextrakts, Konzentrieren des getrockneten Extrakts, Gießen des Extrakts in 3 Volumen Chloroform unter Rühren und Sammeln des Präzipitats, welches durch Wiederholen der Auflösung in Ethylacetat und Präzipitierung mit Chloroform gereinigt werden kann, hergestellt werden. Andere Extraktionsverfahren, die zur gleichen Zusammensetzung des Extrakts führen, können ebenfalls verwendet werden, um diesen Extrakt herzustellen.

Procyanidine normalisieren in vitro die Plättchenaggregation, wobei die Wirkungen mit ASS vergleichbar sind. Die inhibierende Wirkung auf die Plättchenaggregation kann vermutlich durch die Tatsache erklärt werden, daß die Thromboxanbiosynthese durch Procyanidine in zellfreien Systemen und in intakten Plättchen inhibiert wird (Chang, W.-C., Hsu, F.-L.: Inhibition of platelet aggregation and arachidonic acid metabolism in platelets by procyanidins; Prostaglandins, Leukotrienes and essential fatty acids, 38, 181-188 (1989)). Pycnogenol® kann als Mittel für Tiere oder Menschen verwendet werden, um Kapillargefäße abzudichten, so daß die Bildung von Ödemen verhindert wird (Gabór, M., Engi, Etelka, Sonkodi, S.: Die Kapillarwandresistenz und ihre Beeinflussung durch wasserlösliche Flavonderivate bei spontan hypertonischen Ratten; Phlebologie, Schattauer Verlagsgesellschaft mbH 1993, S. 178 bis 182; Schmidtke, 1., Schoop, W.: Das hydrostatische Ödem und seine medikamentöse Beeinflussung, Swiss. Med. 6, Nr. 49 (1984)).

Weiterhin ist es bekannt, das Pycnogenol® als Radikalfänger wirkt und entzündungshemmende Eigenschaften in Tieren aufweist (Blazsó et al., supra). Eine Inhibierung der Plättchenreaktivität in Menschen konnte aus diesen Eigenschaften nicht abgeleitet werden.

Überraschenderweise gelingt es durch die Verabreichung des Rindenextrakts von Pinus Pinaster, die Plättchenaggregation unter normaler Oxygenierung im Gewebe zu hemmen. Das erfindungsgemäß aus dem Rindenextrakt der Strandkiefer hergestellte Mittel hemmt die primäre Plättchenaggregation innerhalb intakter normaler Blutgefäße durch Eingriff in den Arachidonsäure-Metabolismus und hemmt gleichzeitig die durch Adrenalin verursachte Plättchenaggregation. Adrenalin, welches vor allem unter Streß in größeren Mengen gebildet wird, aktiviert Plättchen durch Bindung an adrenergische Rezeptoren und führt zur Plättchenaggregation. Pycnogenol® ist in der Lage, die Adrenalin-induzierte Plättchenaggregation unternormalen Bedingungen zu verhindern, bei denen keine freien Radikale gebildet werden.

Die Erfindung basiert auf der Erkenntnis, daß Pycnogenol® nach oraler Einnahme die durch Rauchen induzierte erhöhte Plättchenreaktivität in Menschen hemmt und daß Pycnogenol® in vitro auch die Adrenalin-induzierte Aggregation von menschlichen Plättchen normalisiert.

Rauchen ist eine weitere Ursache für erhöhte Plättchenaggregation. Rauchen beeinflusst den Metabolismus von Arachidonsäure in einer Weise, dass aggregationsfördernde Substanzen in höheren Mengen und aggregationshemmende Substanzen in geringeren Mengen gebildet werden. Unter normalen physiologischen Bedingungen werden Prostaglandine, Prostacyclin und Thromboxane aus Arachidonsäure durch den Cyclooxygenaseweg gebildet. Prostaglandine wirken der Plättchenaggregation entgegen, während Thromboxane die Plättchenaggregation verstärken. Unter physiologischen Bedingungen befinden sich diese beiden Faktoren in einem Gleichgewicht. Rauchen erhöht die Thromboxanbildung. Weiterhin hemmt Nikotin, welches in Tabakrauch enthalten ist, die Bildung von Prostacyclinen. Deshalb wird während des Rauchens das Gleichgewicht von aggregationsfördernden und aggregationshemmenden Substanzen so gestört, dass die aggregationsinduzierenden Thromboxane vorherrschen. Zudem setzt Rauchen Adrenalin frei, welches ebenfalls eine plättchenaktivierende Substanz darstellt. Die erhöhte Plättchenaggregation nach Rauchen kann deshalb sowohl durch den veränderten Arachidonsäure-Metabolismus mit erhöhter Thromboxan-Bildung als auch durch den erhöhten Ausstoß von Adrenalin bewirkt werden.

Einige der in Pycnogenol® enthaltenen Stoffe, insbesondere Procyanidine und Protocatechusäure, sind in der Lage, die Biosynthese von Thromboxan in vitro zu hemmen. Erfindungsgemäß wird deshalb der Rindenextrakt der Strandkiefer zur Herstellung eines Mittels zur Regelung der Blutplättchenaggregation verwendet, wobei die Hemmwirkung insbesondere auf der Hemmung der Thromboxan-Biosynthese beruht, welche beispielsweise durch Rauchen erhöht wird.

Es ist bekannt, daß Pycnogenol® nur geringe Nebenwirkungen erzeugt, wie etwa gastro-intestinale Beschwerden, wenn der Extrakt auf leeren Magen eingenommen wird. Solche Nebenwirkungen wurden nach Einnahme zusammen mit Mahlzeiten nicht angezeigt.

Das neue Verwendungsverfahren gemäß der vorliegenden Erfindung erlaubtsomit die Normalisierung der erhöhten Plättchenreaktivität ohne die nachteiligen Wirkungen zu erzeugen, die mit der Einnahme von ASS verbunden sind, insbesondere ohne die Blutungszeit zu beeinflussen, da Pycnogenol® keine Erhöhung der Blutungszeit in Menschen - im Gegensatz zu ASS - verursacht. Im Gegensatz zur Dosis von Pycnogenol® von 100 mg täglich, wenn es als Radikalfänger und entzündungshemmendes Mittel eingesetzt wird, beträgt die Dosis für die Normalisierung einer erhöhten Plättchenreaktivität 200 bis 500 mg täglich, bevorzugt etwa 250 bis 350 mg täglich, wobei eine einzelne Dosiseinheit etwa 50 bis 150 mg beträgt. Der Strandkiefern-Rindenextrakt kann in trockener Form, z.B. in Form von Tabletten, beschichteten Pillen, Pellets, Kapseln, Oblatenkapseln oder in Form von Lösungen, welche mit herkömmlichen pharmazeutischen Lösungsmitteln hergestellt werden, verabreicht werden. Übliche pharmazeutisch geeignete Exzipienten, Verdünnungsmittel und Trägerstoffe können ebenfalls verwendet werden.

EP 0 800 827 B1

Die Erfindung wird durch die folgenden Beispiele in Verbindung mit den Figuren der beigefügten Zeichnungen veranschaulicht.

Figur 1 zeigt die Erhöhung der Plättchenreaktivität nach Rauchen ohne die Einnahme von ASS und nach der Einnahme von 500 mg ASS oder 100 mg Pycnogenol®, was die Wirkung von ASS sowie von Pycnogenol® durch die ΔPR-Differenz im Plättchenreaktivitätsindex zu dem Wert vor dem Rauchen veranschaulicht.

Figur 2 zeigt die Blutungszeit nach der Verabreichung von 500 mg ASS und Pycnogenol® und ohne die Verabreichung eines Wirkstoffs.

Figur 3 zeigt die Abnahme der Adrenalin-induzierten Plättchenreaktivität nach Zugabe von Kiefernrindenextrakt in vitro.

**Beispiel 1**

Es wurden Untersuchungen mit 22 männlichen gesunden Rauchern durchgeführt. Die schriftliche Zustimmung sowie die Genehmigung des örtlichen Ethikkommittees wurden eingeholt. 500 mg ASS oder 100 mg Pycnogenol™ (Rindenextrakt der Strandkiefer) wurden als Tabletten 2 bis 3 Stunden vor der ersten Blutprobennahme in Intervallen von 2 Wochen verabreicht. Eine Leerwertuntersuchung wurde zuvor durchgeführt, um einen Untergrundwert zu erhalten. Unmittelbar nach einer ersten Blutprobennahme rauchten die Freiwilligen 3 Zigaretten innerhalb von 30 Minuten. Danach wurde die Blutprobennahme wiederholt. 300 µl Blut wurden jeweils in einer mit EDTA-Puffer gefüllten Spritze und in einer weiteren mit EDTA-Formaldehydpuffer gefüllten Spritze suspendiert. Die roten Blutzellen wurden in beiden Proben gezählt. Während die aneinander anhaftenden oder an rote Blutzellen anhaftenden aktivierten Plättchen in der EDTA-Probe gelöst wurden, blieben sie im EDTA-Formaldehydmedium fixiert. Eine Zentrifugation bewirkte, daß nur die nicht-aggregierten Plättchen im Überstand verblieben. Die Plättchen wurden in beiden Überständen mittels eines Plättchenzählers gezählt. Der Plättchenreaktivitätsindex (PR) wurde wie folgt berechnet:

$$\frac{\text{Plättchen in EDTA x rote Blutzellen in Formalin-EDTA}}{\text{Plättchen in Formalin-EDTA x rote Blutzellen in EDTA}} = \text{PR}$$

Der PR steigt linear mit der Anzahl von aggregierten Plättchen.

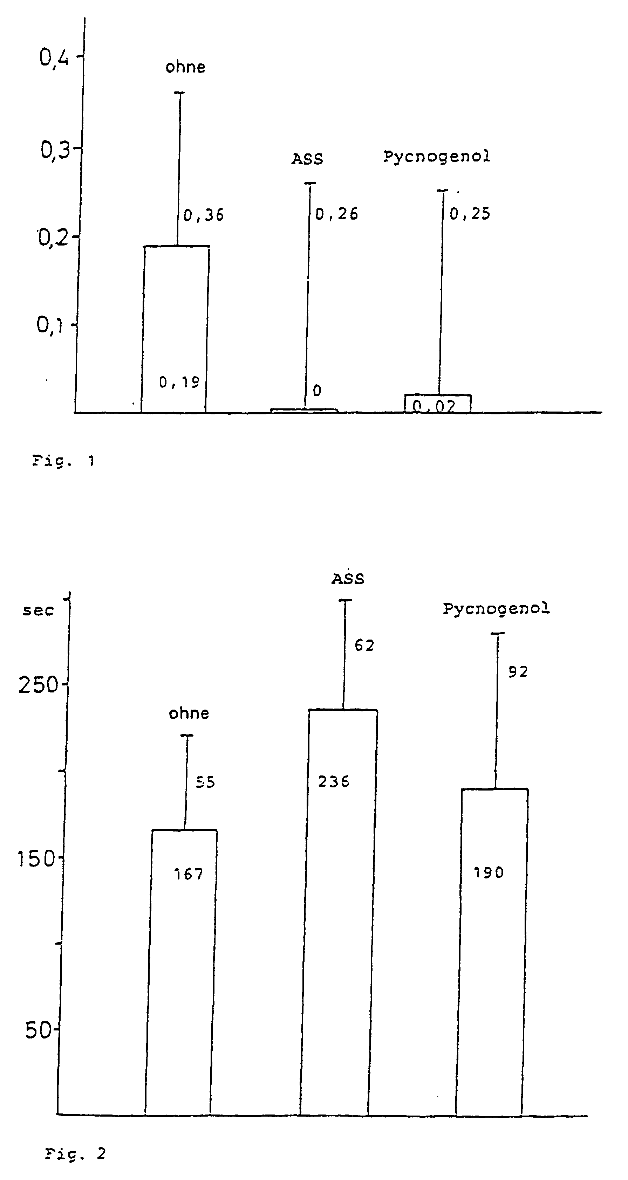

Figur 1 zeigt die erwartete Erhöhung der Plättchenreaktivität nach Rauchen mit Standardabweichungen. Die durch Rauchen induzierte Plättchenaggregation wurde durch ASS (p = 0,06) und durch den Kiefernrindenextrakt (p = 0,08) signifikant normalisiert.

Daraus folgt, daß eine einzelne Dosis von 100 mg Pycnogenol® in der Lage ist, die durch Rauchen induzierte Plättchenaggregation im gleichen Ausmaß wie 500 mg ASS zu normalisieren. Der geringe Unterschied zwischen beiden Behandlungen ist nicht signifikant.

Die Blutungszeit wurde nach Punktieren des Ohrläppchens vor dem Rauchen bestimmt. Figur 2 zeigt, daß Blutungszeiten ohne Verabreichung eines Arzneiwirkstoffes und nach Einnahme des Kiefernrindenextrakts nicht signifikant unterschiedlich sind. Nach Einnahme von ASS ist die Blutungszeit jedoch signifikant länger (p = 0,002). Die Blutungszeit nach Einnahme von 100 mg Pycnogenol® ist beträchtlich kürzer als nach Einnahme von 500 mg ASS (p = 0,02).

Dieses Beispiel veranschaulicht, daß Pycnogenol® die Plättchenaggregation bei einer geringeren Dosis im Vergleich zu ASS normalisiert, ohne jedoch die Blutungszeit zu beeinflussen.

**Beispiel 2**

Die folgende Untersuchung wurde ausgeführt, um zu bestimmen, ob Pycnogenol® in geeigneten Konzentrationen verwendet werden kann, um Adrenalin-induzierte Plättchenaggregation zu hemmen. Blutproben wurden von gesunden Freiwilligen gesammelt und Thrombozyten-angereichertes Plasma wurde durch wiederholte Zentrifugation erhalten. Die Aggregationsgeschwindigkeit wurde durch Turbidimetrie mit und ohne Vorinkubation mit Pycnogenol® in unterschiedlichen Konzentrationen nach Zugabe von Adrenalin gemessen. Die Hemmgeschwindigkeit wurde wie unten angegeben berechnet.

4

$$\text{Hemmgeschwindigkeit} = \frac{\text{Aggregationsgeschwindigkeit Leerwert} - \text{Aggregationsgeschwindigkeit Kiefernrindenextrakt}}{\text{Aggregationsgeschwindigkeit Kiefernrindenextrakt}} \times 100$$

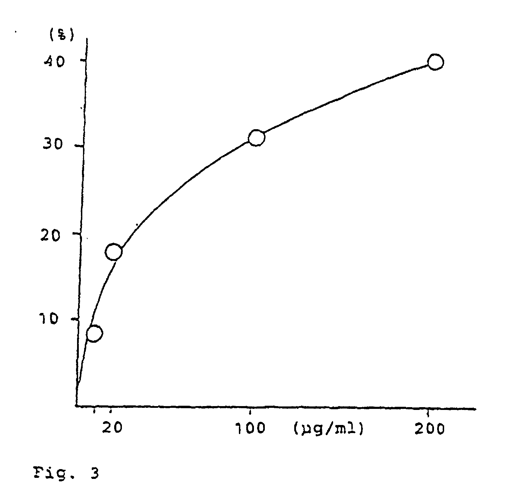

Das Beispiel (Figur 3) zeigt, daß Pycnogenol™ die Plättchenaggregation selbst in Gegenwart von Adrenalin signifikant hemmt.

**Patentansprüche**

1. Verwendung eines Rindenextrakts der Strandkiefer umfassend Procyanidine zur Herstellung eines Mittels zur Regelung der Blutplättchen-Aggregation beim Menschen unter normaler Oxygenierung im Gewebe durch Hemmung der durch Rauchen oder/und Thromboxan oder/und Adrenalin induzierten Plättchenaggregation, wobei ein Mittel zur Verabreichung von 200 bis 500 mg des Rindenextrakts der Strandkiefer pro Tag hergestellt wird.

2. Verwendung nach Anspruch 1,
   **dadurch gekennzeichnet,**
   **daß** ein Mittel zur Verabreichung von 250 bis 350 mg des Rindenextrakts der Strandkiefer pro Tag hergestellt wird.

3. Verwendung nach Anspruch 1 oder 2,
   **dadurch gekennzeichnet,**
   **daß** ein Mittel zur oralen Verabreichung hergestellt wird.

4. Verwendung nach einem der Ansprüche 1 bis 3,
   **dadurch gekennzeichnet,**
   **daß** ein Mittel zur Verabreichung in einzelnen Dosiseinheiten von 50 bis 150 mg hergestellt wird.

**Claims**

1. Use of an extract from the bark of the maritime pine containing procyanidins to produce an agent for regulating blood platelet aggregation in humans under normal tissue oxygenation by inhibiting platelet aggregation induced by smoking or/and thromboxane or/and adrenaline, wherein an agent is produced for administering 200 to 500 mg of the bark extract of the maritime pine per day.

2. Use as claimed in claim 1,
   wherein
   an agent is produced for administering 250 to 350 mg of the bark extract of the maritime pine per day.

3. Use as claimed in claim 1 or 2,
   wherein
   an agent is produced for oral administration.

4. Use as claimed in one of the claims 1 to 3,
   wherein
   an agent is produced for administration in single dose units of 50 to 150 mg.

**Revendications**

1. Utilisation d'un extrait d'écorce de pin maritime comprenant de la procyanidine pour la fabrication d'un agent pour la régulation de l'agrégation des plaquettes sanguines chez l'homme sous oxygénation normale dans le tissu par inhibition de l'agrégation des plaquettes induite par le tabagisme ou/et le thromboxan ou/et l'adrénaline, moyennant quoi un agent pour l'administration de 200 à 500 mg de l'extrait d'écorce de pin maritime par jour est fabriqué.

2. Utilisation selon la revendication 1, **caractérisée en ce qu'**un agent pour l'administration de 250 à 350 mg de l'extrait d'écorce de pin maritime par jour est fabriqué.

3. Utilisation selon la revendication 1 ou 2, **caractérisée en ce qu'**un agent pour l'administration orale est fabriqué.

4. Utilisation selon l'une des revendications 1 à 3, **caractérisée en ce qu'**un agent pour l'administration en doses individuelles de 50 à 150 mg est fabriqué.

Fig. 1

Fig. 2

Fig. 3